# EUROPEAN PATENT APPLICATION

(11) **EP 1 175 896 A1**
(43) Date of publication of application: **30.01.2002**
(21) Application number: 01104674.5
(22) Date of filing: 24.02.2001
(51) Int. Cl.: A61K 7/48, A61K 7/06, A61K 7/50

(54) **Cosmetic composition suitable for sensitive and reactive skin**

(30) Priority: 28.07.2000 IT MI001730
(71) Applicant: ICIM Istituto Chimico Italiano Milano SRL, 20024 Garbagnate Milanese (MI) (IT)
(72) Inventor: Agostinini, Paolo, ICIM Istituto Chimico Italiano, 20024 Garbagnate Milanese (MI) (IT); Pecis, Laura, ICIM Istituto Chimico Italiano, 20024 Garbagnate Milanese (MI) (IT)
(74) Representative: Beneduce, Gianna

(57) **Abstract**

A cosmetic composition particularly suitable for sensitive and reactive skins, characterized by the fact that it is free from preservatives and/or practically free from nickel.

## Description

The present invention relates to a cosmetic composition free from preservatives and practically free from nickel, suitable to be applied to particularly reactive skins or skins made sensitive by irritating external agents.

It is known that contact allergic dermatitis (CAD), which affects women more frequently than men, causing irritating epidermis alterations such as reddening, itching and scaliness and, in the most severe cases, ulcerations, may frequently be caused by the use of cosmetics.

It is also known that nickel and preservative substances contained in cosmetics, are often responsible for the onset of contact dermatitis.

Nickel represents the main cause of skin sensitization or contact dermatitis and the high frequency of said pathology is undoubtedly due to its intrinsic characteristics and to its wide environmental diffusion; it can consequently be considered as an ubiquitary hapten. When in contact with the skin, nickel spreads towards the cutis with perspiration at a speed that is proportional to the temperature and to the time of the contact.

In the past the safety of cosmetics was based on the knowledge of their ingredients; today the development of new analytical technologies and of more and more refined instruments have made it possible to identify even traces of other substances which may be present as impurities in the ingredients themselves. With these new techniques it has been possible to demonstrate the nearly constant presence of nickel in cosmetics, even in such concentrations, as to cause sensitization to the metal, resulting in symptoms of contact dermatitis.

The cosmetic composition object of the invention, tested for its the nickel concentration, provided values around 0.01 ppm on an average, and in no case higher than the concentration of 0.1 ppm. Said composition is therefore particularly suitable for use in persons suffering from contact allergic dermatitis and in persons having a particularly reactive skin, to whom even traces of nickel in a cosmetic, could cause the onset of redness and/or itching.

The analytical method utilized for the nickel determination in the composition of the invention is based on hot digestion with nitric acid and perchloric acid using the following procedure. Place a sample of 4g in a 200mL flask with a frosted glass neck, add 20mL of concentrated nitric acid and place the air-cooler on the flask; heat on a heating plate to the temperature of about 200°C for about one hour, and in any case until all brown fumes are abated. If the sample contains many solvents or if, after the addition of nitric acid, brown fumes are copiously evolved, cool the sample for about one hour then proceed with caution to the next heating. When the brown fumes disappear cool the sample. Add a further 10mL of concentrated perchloric acid and heat for at least one hour until the brown fumes disappear. Leave to cool and add 20mL of acidulated water (5% HNO₃). Bring to boiling point in order to aid solubility, then leave to cool and filter on a fast folded filter in a 100mL calibrated beaker, carefully washing the flask and bringing to volume with distilled water.

The resulting solution is analysed by atomic absorption according to the procedures indicated for the used apparatus. As is well known cosmetic products are an optimal culture medium for bacteria as well as for fungi and yeasts and it is therefore necessary that, in order to maintain their characteristics and biological stability, preservatives have to be added to them, i.e. chemical compounds having antibacterical activity. Due to their high reactivity, these preservatives may lead, in the treated area, to the onset of contact dermatitis and, occasionally, of contact irritant dermatitis.

The preservatives which are most active on the micro-organisms are also the most toxic ones for cells of skin tissues. A badly preserved cosmetic may lead to uncontrolled growth of micro-organism with consequent formation of pharmacologically active bacterial metabolic products which can cause considerable damage to the treated skin. The presence of a preservative in a cosmetic product, even if not pleasant, is therefore to be considered necessary.

The object of the present invention consists in providing a cosmetic composition practically free from nickel and/or totally free from those traditional preservatives used in the field and classified as such by the regulation in force (Law 11.10.86 n. 713 "Norms for the application of directives of the European Economic Community on the production and the sale of cosmetics").

A further object of the present invention consists in the preparation process to obtain a cosmetic composition practically free from nickel and/or totally free from preservatives traditionally used in the field.

It has now been found that by utilizing specific suitable compounds in cosmetic formulations such as surfactants, oxidizing agents, moistening agents, esters, polymers, acids and vegetable extracts, mixed together in suitable conditions and proportions, it is possible to create an environment totally unsuitable to the microbial development, i.e. able to reduce their possibilities of survival. Said specific mixture therefore represents a valid alternative to antibacterial substances traditionally used in the cosmetic field.

More particularly, said self-preserving mixture, in suitable conditions and ratios, contains two or more substances included in the group formed by:
- esters of aliphatic or aryl organic acids, in the amount of 0-6%;
- saturated fatty acids containing 6-15 carbon atoms and unsaturated fatty acids containing 16-18 carbon atoms, in the amount of 0-10%;
- aminoacyl derivatives, for example, glycine, hydroxyproline, arginine, phenylalanine, lysine and their derivatives, optionally conjugated with suitable fatty acids, for example, palmitic, octanoic and undecylenic acid, in the amount of 0-3%;
- moistening substances, for example, propylene, butylene and pentylene glycol, polyglycols, glycerine and its derivatives, in the amount of 0-30%;
- vegetable derivatives, for example, titrated extracts and/or active principles and/or essential oils from plants belonging to the caprifoliaceae, liliaceae, myrtaceae, asteraceae (compositae) and rutaceae families, in the amount of 0-2%;
- anionic surfactants, for example, sodium sarcosinate, sodium dodecylsulphate, alkylarylsulphonates, in the amount of 0-30%;
- amphoteric surfactants, for example, alkylamidopropylbetaine, alkylimidazoline, alkylaminopropionates, in the amount of 0-10%; and
- cross polymers having osmotic activity such as, for example, (C₁₀-C₃₀)alkylcopolymer acrylates, in the amount of 0-20%, and it provides, in cosmetic formulations, a satisfactory protection against microbiological alterations covering the wide microbial spectrum (gram+; gram-; moulds and yeasts).

The cosmetic composition of the invention is conveniently formulated as an ointment, paste, fluid and/or creamy emulsion, powder, lotion, gel, oil, solution, soap, foam and shampoo.

According to the present invention the raw materials for the preparation of the cosmetic composition are weighed and, depending on their hydrophylic or lipophylic nature, are placed in suitable, specific, AISI 316 steel containers with the surface perfectly specular and then heated and/or cooled, mixed and/or whirled in order to get the finished products.

The chemically and microbiologically pure deionized water, used in the production process, is supplied from a reverse osmosis plant with an absolute filter located downstream of it. At the end of the production process, the semi-manufactured product is transferred by means of pumps to the AISI 316 steel containers with the surface perfectly specular, provided with a suitable sealing lid. Said containers are then transferred to the packaging department, then the product is conveniently dosed and split into the sale packages.

The following examples have the purpose to better clarify the invention without however limiting it.

### Production scheme of an emulsion

Phase 1. Place the raw materials, which represent the fatty emulsion phase, in a AISI 316 specular surface steel melting vessel and thermostat at the fixed temperature.

Phase 2. Place a portion of the deionized water and the thermostable and hydrosoluble raw materials in a AISI 316 steel turboemulsifying apparatus having a specular surface, and thermostat to the fixed temperature. When the fixed temperature is reached, add any necessary polymers, which need a suitable hot homogenization, stirring according to fixed times and speed, and continuously heating.

Phase 3. Prepare the aqueous solutions of the remaining active compounds separately in AISI 316 steel containers, taking into consideration any thermostability and incompatibilities.

Phase 4. When the fixed temperature is reached, add phase 1 to phase 2, operating under vacuum with the homogenization turbines, mixer and counter-mixer according to fixed times and speed.

Phase 5. Slowly cool and, during this phase, add the solutions of phase 3.

Phase 6. Stop cooling, switch off the mixer and the countermixer and carry out analytical controls on the semifinished product.

### Production scheme of a detergent.

Phase 1. Place the primary surfactants, which need heating, in a AISI 316 steel specular surface mixer and add a portion of the total amount of water foreseen for the formulation. Operate the mixer and the counter-mixer at minimum speed up to complete dissolution.

Phase 2. Add a further portion of water and start cooling.

Phase 3. Prepare solutions of any active ingredients separately in suitable containers and separately add to phase 2.

Phase 4. Continue mixing and cooling until the final product is obtained.

### EXAMPLES 1-4

According to the above described procedures, using AISI 316 steel apparatus the following cosmetic compositions are prepared.

| Fluid emulsion | |
|---|---|
| paraffin oil | 11.00 |
| glycerine | 5.00 |
| polysorbate 90 | 4.00 |
| sorbitan stearate | 2.50 |
| pentylene glycol | 1.00 |
| polyquaternium-7 | 1.00 |
| sarcosine | 0.50 |
| E.D.T.A. | 0.20 |
| water | q.s. ad 100.00 |

| Shampoo | |
|---|---|
| ethoxylated lauryl alcohol | 25.50 |
| polyglycol | 16.00 |
| paraffin oil | 12.00 |
| sulphosuccinate | 6.00 |
| sodium sarcosinate | 4.00 |
| glycerine | 2.00 |
| polyquaternium-7 | 1.70 |
| parfum | 0.50 |
| water | q.s. ad 100.00 |

| Creamy emulsion | |
|---|---|
| cyclomethicone | 10.00 |
| ethoxylated C₆-C₁₂ acids | 8.00 |
| paraffin oil | 7.00 |
| C₁₂-C₁₅ alkylbenzoate | 3.00 |
| ethoxylated cetylstearyl alcohol | 2.40 |
| jojoba oil | 2.00 |
| stearin | 1.00 |
| phenyltrimethicone | 1.00 |
| ethylhexylglycerine | 1.00 |
| polyacrylic acid sodium salt | 0.75 |
| Caprifoliaceae extract | 0.50 |
| E.D.T.A. | 0.10 |
| water | q.s. ad 100.00 |

| Fluid emulsion | |
|---|---|
| stearyl eptanoate | 5.00 |
| paraffin oil | 6.00 |
| polysorbate 60 | 4.00 |
| glycerin | 3.00 |
| sorbitan stearate | 2.50 |
| cetylstearyl alcohol | 2.00 |
| ethoxylated lauryl alcohol | 1.00 |
| pentylene glycol | 1.00 |
| polyquaternium-7 | 2.00 |
| polyacrylic acid sodium salt | 0.90 |
| α-bisabolole (Asteraceae) | 0.50 |
| capryloyl glycine | 0.50 |
| methylglycine | 0.50 |
| E.D.T.A. | 0.10 |
| water | q.s. ad 100.00 |

## Claims

1. A cosmetic composition free from preservative substances and practically free from nickel, suitable to be applied to sensitive and particularly reactive skins, **characterized by** the fact that it contains a mixture of two or more compounds selected from a group consisting of surfactants, antioxidants, moistening agents, esters, polymers, acids and vegetable extracts, mixed together in suitable proportions and conditions and that it contains less than 0.1ppm of nickel.

2. A cosmetic composition according to claim 1, **characterized by** the fact that the average quantity of nickel contained therein is 0.01 ppm.

3. A cosmetic composition according to claims 1 and 2, **characterized by** the fact that the compounds forming said mixture are selected from a group consisting of: aliphatic or aryl organic acid esters in the amount of 0-6%; saturated fatty acids containing 6-15 carbon atoms and unsaturated fatty acids containing 16-18 carbon atoms in the amount of 0-10%-; aminoacyl derivatives and their derivatives optionally conjugated with suitable fatty acids in the amount of 0-3%; glycols, polyglycols, glycerine and its derivatives in the amount of 0-30%; vegetable derivatives of plants belonging to the caprifoliaceae, liliaceae, mirtaceae, asteraceae and rutaceae families in the amount of 0-2%; anionic surfactants in the amount of 0-30%; amphoteric surfactants in the amount of 0-10% and cross polymers with osmotic activity in the amount of 0-20%.

4. A cosmetic composition according to claim 3, **characterized by** the fact that the aminoacyl derivatives are selected from a group consisting of glycine, hydroxyproline, arginine, phenylalanine, lysine and their derivatives, the fatty acids are selected from palmitic, octanoic and undecylenic acids; glycols are selected among propylene, butylene and pentylene glycol; vegetable extracts may be titrated extracts, active principles, essential oils optionally mixed together; anionic surfactants are selected from sodium sarcosinate, sodium dodecylsulphate, alkylarylsulphonates; amphoteric surfactants are selected from alkylamidopropylbetaine, alkylimidazoline, alkylaminopropionates; and cross polymers with osmotic activity are (C₁₀-C₃₀)alkylcopolymer acrylates.

5. A cosmetic composition practically free from nickel, suitable to be applied to sensitive and particularly reactive skins, **characterized by** the fact that it contains less than 0.1ppm of nickel.

6. A cosmetic composition free from preservatives suitable to be applied to sensitive and particularly reactive skins, **characterized by** the fact that it contains a mixture of two or more compounds selected from the group consisting of surfactants, antioxidants, moistening agents, esters, polymers, acids and vegetable extracts mixed together in suitable proportions and conditions.

7. Process for the preparation of the cosmetic composition of claim 1, according to the disclosure.
